# EUROPÄISCHE PATENTSCHRIFT

(11) **EP 2 858 695 B1**
(45) Veröffentlichungstag und Bekanntmachung des Hinweises auf die Patenterteilung: **08.08.2018**
(21) Anmeldenummer: 13730800.3
(22) Anmeldetag: 04.06.2013
(51) Int. Cl.: A61M 1/16, B65D 75/00, B65D 33/02

(54) **KONZENTRATBEHÄLTNIS MIT STÜTZELEMENTEN**
CONCENTRATE RECEPTACLE WITH SUPPORT ELEMENTS
CONTENANT POUR CONCENTRÉ DOTÉ D'ÉLÉMENTS DE SUPPORT

(30) Priorität: 06.06.2012 DE 102012011250; 06.06.2012 US 201261656309 P
(43) Veröffentlichungstag der Anmeldung: 15.04.2015
(73) Patentinhaber: Fresenius Medical Care Deutschland GmbH, 61352 Bad Homburg (DE)
(72) Erfinder: BALSCHAT, Klaus, 97525 Schwebheim (DE); GAGEL, Alfred, 96123 Litzendorf (DE); HÜMMER, Dirk, 97520 Hirschfeld (DE); KLÖFFEL, Peter, 97720 Nüdlingen (DE); NICHOLAS, Olaf, 97318 Kitzingen (DE); STÄBLEIN, Tilman, 97072 Würzburg (DE)
(74) Vertreter: Schön, Andrea
(86) Internationale Anmeldenummer: PCT/EP2013/001623
(87) Internationale Veröffentlichungsnummer: WO 2013/182293

(56) Entgegenhaltungen:
- WO-A1-96/01775
- WO-A1-2005/120972
- WO-A1-2011/115270
- WO-A2-2007/144427
- GB-A- 2 311 753
- US-A1- 2009 297 741

## Beschreibung

### Zusammenfassung

Die Erfindung betrifft einen Behälter zur Bereitstellung von Dialysekonzentrat mit integrierten Stützelementen.

### Stand der Technik

In der EP 1 344 550 und der DE 10 2011 106 248.7 werden Behälter zur Bereitstellung von Konzentraten für die Dialyse beschrieben. Behälter mit Stützelementen sind zum Beispiel aus der WO 2005/120972 bekannt.

### Problemstellung

Bei der Therapie von Nierenversagen durch Hämodialyse werden pro Behandlung circa 1801 Dialyselösung benötigt. Um den Aufwand bei Transport und Lagerung dieser Produkte möglichst gering zu halten, erfolgt die Lieferung üblicherweise in Form von Konzentraten, wobei aus Stabilitätsgründen das basische und das saure Konzentrat getrennt bereit gestellt werden.

Das basische Konzentrat wird üblicherweise als Trockenkonzentrat bestehend aus reinem Natriumbicarbonat geliefert. Daraus wird von der Dialysemaschine im Durchfluss eine gesättigte Lösung hergestellt, was bedeutet, dass während der Prozedur immer ein Anteil an ungelöstem Salz in dem Behälter vorhanden ist. Wasser wird kontinuierlich zugeführt und die gesättigte Lösung kontinuierlich entnommen. Das Volumen des Behälters für das basische Konzentrat entspricht damit ungefähr dem Volumen des für eine Behandlung benötigten Salzes (circa 0,5-11).

Das saure Konzentrat wird meist als Flüssigkonzentrat geliefert. Es besteht hauptsächlich aus Kochsalz und enthält in weitaus geringeren Mengen weitere Elektrolyte wie Magnesium-, Calcium- und Kaliumchlorid, die Säure und eventuell weitere Zusätze wie z.B. Glucose. Wird das saure Konzentrat als Trockenkonzentrat geliefert, kann es nicht wie das basische Konzentrat im Durchfluss hergestellt werden. Besonders die Konzentrationen der nur geringfügig vorhandenen Komponenten wie Kalium und Calcium müssen genau eingehalten werden, um eine für den Patienten sichere Dialyselösung zu erhalten. Das gesamte Trockenkonzentrat im Behälter muss vor Verdünnung zur Dialyseflüssigkeit in Lösung gebracht werden. Damit dies möglich ist, muss der Behälter für das saure Konzentrat ein deutlich größeres Volumen (circa 4-121) bereitstellen, als der Behälter für das basische Konzentrat. Für den Transport und die Lagerung soll der Behälter nur ein möglichst geringes Volumen beanspruchen. Dafür besonders geeignet ist ein Beutel, der auf eine möglichst geringe Größe gefaltet werden kann, und sich bei Befüllung mit Flüssigkeit entfaltet.

Liegt das Konzentrat in trockener Form vor, muss dem Behälter zur Auflösung des Trockenkonzentrates Flüssigkeit durch die Dialysemaschine zugeführt und nach Herstellung des Flüssigkonzentrates entnommen werden. Deshalb muss der Behälter flüssigkeitsdicht mit einem Verbinder an der Dialysemaschine anbracht werden. Die Behältnisse werden über ein Verbindungsmittel an die Konnektoren der Dialysemaschine angehängt. Haben die Behälter ein größeres Volumen und damit auch ein größeres Gewicht ergibt sich eine Zugbelastung an den entsprechenden Konnektoren.

Aufgabe der vorliegenden Erfindung ist es flexible Behälter für Konzentrate zur Verfügung zu stellen, die für Transport und Lagerung möglichst klein und leicht sind. Bei Benutzung sollen diese ein größeres Volumen zur Verfügung stellen, sie sollen eine größere Stabilität aufweisen und die Zugbelastung an der Dialysemaschine soll weitgehend vermieden werden.

Nach der Lehre der vorliegenden Erfindung wird diese Aufgabe gelöst durch einen Behälter nach Anspruch 1, der Verwendung des Behälters nach Anspruch 6, einem Dialysegerät nach Anspruch 7 und einem Verfahren nach Anspruch 10. Vorteilhafte Ausgestaltungen sind Grundlage der Unteransprüche.

### Zusammenfassung der Erfindung

Die Erfindung betrifft einen Behälter für ein Konzentrat, das zur Herstellung einer Dialyselösung geeignet ist. Dieser Behälter weist ein Verbindungsmittel auf, mittels dessen der Behälter an ein Dialysegerät oder eine Zubereitungseinheit für Flüssigkonzentrate konnektiert werden kann. Durch das Verbindungsmittel werden zwei Fluidverbindungen zwischen dem Dialysegerät und dem Behälter hergestellt. Durch eine dieser Fluidverbindungen ist die Zufuhr einer Flüssigkeit, vorzugsweise Wasser, oder die Zufuhr eines Gases, vorzugsweise Luft, möglich. Auch die gleichzeitige Zufuhr von Flüssigkeit und Gas ist möglich. In einer bevorzugten Ausführungsform dient diese Fluidverbindung auch zur Entnahme des Konzentrates aus dem Behälter. Die zweite Fluidverbindung kann zur Entlüftung des Behälters verwendet werden. Diese Fluidverbindung kann weiterhin ein Rückschlagventil enthalten. Dieses Ventil kann sich bei Erreichen eines bestimmten Innendrucks in dem Behälter öffnen. Dadurch wird ein zu hoher Überdruck im Behälter vermieden. Des Weiteren enthält der Behälter integrierte Stützelemente. Diese Stützelemente sind so ausgestaltet, dass sie vor Verwendung des Behälters an einer Dialysemaschine oder einer Zubereitungseinheit das Volumen und das Gewicht des Behälters möglichst wenig vergrößern. Sie erhöhen deshalb kaum den Aufwand für Transport und Lagerung des Produkts. Erst bei der Zufuhr von Flüssigkeit und/ oder eines Gases in den Behälter prägen die Elemente ihre Stützfunktion aus. Dabei ist das Stützelement ein Luftpolster. Dieses Luftpolster wird durch die Zufuhr von Luft gefüllt. Durch die Wahl des Ventils in der Entlüftungsleitung ist der Aufbau eines Drucks in dem Behälter möglich, der zur Füllung des Luftpolsters verwendet werden kann.

Das Luftpolster bildet dabei ein Außenbeutel um den Konzentrat-gefüllten Innenbeutel, welcher diesen größtenteils umschließt und so stabilisiert. Das Luftpolster kann auch aus mehreren Kompartimenten bestehen. Der Außenbeutel kann dabei so ausbildet sein, dass der Behälter nach Füllung des Luftpolsters selbststehend ist. In einer bevorzugten Ausführungsform sind Innen- und Außenbeutel über ein Rückschlagventil verbunden. Dann kann der Außenbeutel durch Zufuhr von Gas über die Fluidleitung zur Dialysemaschine in den Innenbeutel gefüllt werden. Das Gas wird dann über ein Einführmittel in den Innenbehälter geleitet und gelangt über das Rückschlagventil in den Außenbeutel. Ist der Außenbeutel gefüllt, kann überschüssiges Gas durch ein Entlüftungsmittel im Innenbeutel abgeleitet werden.

Alternativ kann der Außenbeutel auch mit einem separaten Zugang ausgestattet sein, über den ein Gas zugeführt werden kann.

In einer alternativen Ausführungsform kann das Entlüftungsmittel als gefalteter, mit dem Behälter verbundener Fuß ausgebildet sein. Dieser entfaltet sich bei Füllung des Behälters mit Flüssigkeit und/oder Luft und prägt so seine Stützfunktion aus. In einer bevorzugten Ausführungsform ist der Fuß so ausgestaltet, dass der Behälter selbststehend ist.

In einer bevorzugten Ausführungsform ist das im Behälter enthaltene Konzentrat ein Trockenkonzentrat. Handelt es sich bei dem Trockenkonzentrat um das saure Konzentrat, muss, wie schon oben beschrieben, das gesamte Konzentrat in Lösung gebracht werden, bevor es zur Herstellung einer Dialyselösung verwendet werden kann. Die Menge an Salz im Behältnis muss genügen, um mindestens eine Dialysebehandlung durchzuführen. Zudem sind die Verdünnungsverhältnisse in der Maschine zur Herstellung der Dialyselösung festgelegt, weshalb der Behälter ein bestimmtes Volumen bereitstellen muss. Der Behälter hat dann nachdem er mit einer Flüssigkeit, vorzugsweise Wasser, auf seine Sollkonzentration befüllt wurde ein Volumen zwischen 4 und 151.

Ein Behälter für Trockenkonzentrat muss zudem einen Bereich aufeinander zu gewandte Wandbereiche aufweisen, zwischen denen sich ein talförmiger Bereich oder eine Vertiefung ausbildet, wobei das Trockenkonzentrat in der Betriebsposition des Behälters zumindest auch in dem talförmigen Bereich bzw. in der Vertiefung vorliegt. In diesen Bereich ragt dann auch das Einführmittel für Flüssigkeit, vorzugsweise Wasser, und/oder Gas, vorzugsweise Luft, hinein. Damit wird die Auflösung des Konzentrates, wie in der DE 10 2011 106 248.7 beschrieben, optimiert. Dadurch können aber gängige Behälter wie Standbeutel nicht benutzt werden. Spezielle erfindungsgemäße Stützelemente sind dann besonders vorteilshaft.

Die Erfindung betrifft ferner die Verwendung eines erfindungsgemäßen Behälters zur Herstellung eines Konzentrates, insbesondere eines sauren Flüssigkonzentrates, zur Herstellung einer Dialyselösung, vorzugsweise einer Dialyselösung für die Hämodialyse.

Die vorliegende Erfindung betrifft des Weiteren ein Dialysegerät oder eine Zubereitungseinheit zur Herstellung eines Dialysekonzentrates, welche mit einem erfindungsgemäßen Behälter konnektiert sind Dabei wird das Verbindungsmittel des Behälters mit zwei Fluidleitungen in der Dialysemaschine bzw. der Zubereitungseinheit für Dialyselösungen verbunden.

Über eine Fluidleitung wird eine Flüssigkeit, vorzugsweise Wasser,, und gleichzeitig ein Gas, vorzugsweise Luft, in den Behälter geleitet.

Ist Flüssigkonzentrat in einer geeigneten Konzentration in dem Behälter enthalten, kann durch die Einleitung eines Gases ein Stützelement in Form eines Luftpolsters so gefüllt werden, dass dieses eine weitgehend feste Form annimmt, und so den flexiblen Behälter stabilisiert.

Das Dialysegerät kann dabei Vorrichtungen aufweisen, mittels derer man die Flüssigkeit, vorzugsweise Wasser, und/oder ein Gas, vorzugsweise Luft, aus dem Behälter abführen kann. In der Fluidleitung des Dialysegerätes, die mit dem Entlüftungsmittel des Behälters verbunden ist, können zudem Ventile enthalten sein, die zur Herstellung eines gewissen Drucks in dem Behälter verwendet werden können. Dazu kann dann in der Entlüftungsleitung des Dialysegerätes, die mit der Entlüftungsleitung des Behälters verbunden ist, ein Druckmessgerät vorgesehen sein. So kann das Ventil so lange geschlossen sein, bis das Luftpolster gefüllt ist. Dies kann über das Erreichen eines bestimmten Druckwertes überprüft werden. Muss dann zur Durchmischung des Konzentrates weiteres Gas, vorzugsweise Luft, in den Behälter eingeführt werden, kann das Ventil geöffnet werden. Es entsteht kein zu hoher Druck in dem Behälter. Ist das Luftpolster über ein Rückschlagventil mit dem Behälterkompartiment für das Konzentrat verbunden, bleibt der im Luftpolster aufgebaute Druck erhalten.

Ferner kann das Dialysegerät oder die Zubereitungseinheit wenigstens eine Steuer- oder Regeleinheit aufweisen, die derart ausgeführt ist, dass sie die Zufuhr von Flüssigkeit, vorzugsweise Wasser, und/oder Gas, vorzugsweise Luft, in den Behälter und/oder die Abfuhr von Gas, vorzugsweise Luft und oder eines Flüssigkonzentrats aus dem Behälter steuert oder regelt.
Weiterhin kann diese Steuer- und Regeleinheit so konfiguriert sein, dass sie Signale des Druckmessgerätes verwendet, um das Schließen und Öffnen des Ventiles in der Entlüftungsleitung des Dialysegerätes zu steuern.

Die Erfindung betrifft weiterhin ein Verfahren zur Bereitstellung eines stabilen Behälters für Konzentrate für die Dialyse, der mit einem Dialysegerät oder einer Zubereitungseinheit zur Herstellung von Dialysekonzentraten konnektiert wird. Bei Zufuhr einer Flüssigkeit, vorzugsweise Wasser, und Zufuhr eines Gases, vorzugsweise Luft, erfolgt die Ausprägung einer Stützfunktion durch in dem Behälter integrierte Stützelemente, wobei ein Luftpolster gefüllt wird, das den Behälter stabilisiert.

Eine alternative Möglichkeit ist, dass durch die durch die Flüssigkeitszugabe verursachte Volumenvergrößerung des Behälters zum Aufklappen eines zuvor gefalteten mechanischen Stützelementes führt und der Behälter so stabilisiert wird.

Weitere Einzelheiten und Vorteile der Erfindung werden anhand der in den Zeichnungen dargestellten Ausführungsbeispiele näher beschrieben. Sie zeigen:
Figur 1: Schematische Ansicht eines Behälters für Konzentrate nach dem Stand der Technik
Figur 2: Schematische Vorderansicht eines erfindungsgemäßen Behälters, mit gefülltem Luftpolster, teilweise mit Flüssigkonzentrat gefüllt.
Figur 3: Perspektivische Rückansicht eines erfindungsgemäßen Behälters, mit gefülltem Luftpolster.
Figur 4: Schematische Vorderansicht eines erfindungsgemäßen Behälters, der an einer Dialysemaschine montiert ist.
Figur 5: Perspektivische Ansicht eines erfindungsgemäßen Behälters mit entfaltetem Stützelement.

### Beschreibung

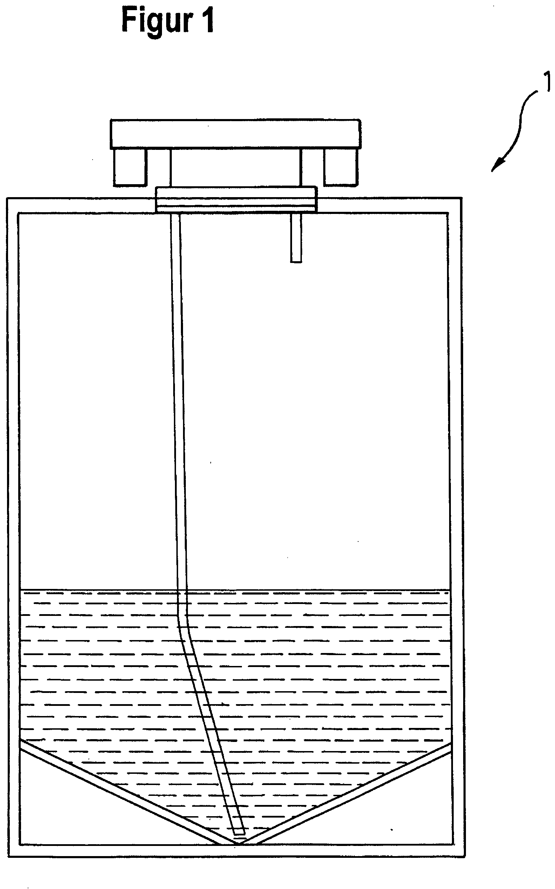

In Figur 1 wird ein Behälter 1 nach dem Stand der Technik gezeigt.

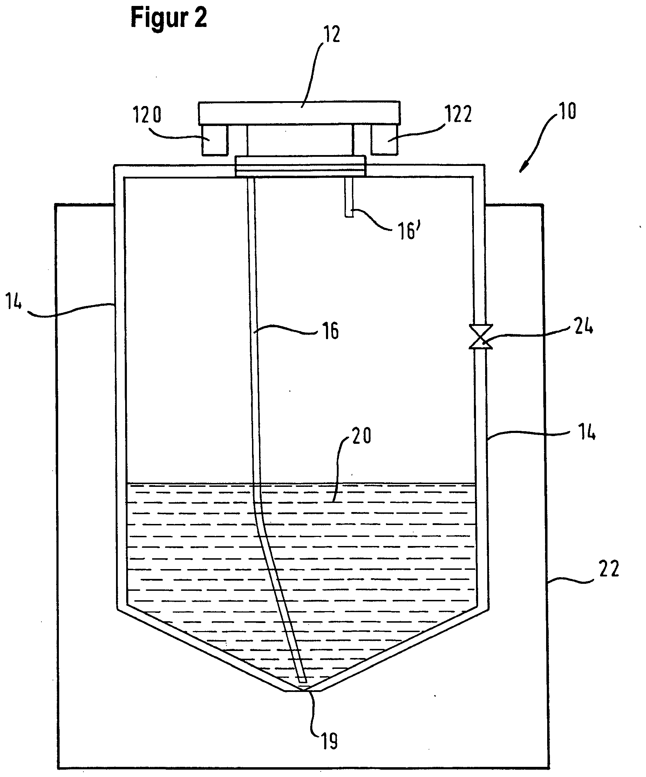

In Figur 2 ist ein erfindungsgemäßer Behälter 10 dargestellt. Er ist als Innenbeutel 14 mit flexiblen Wandungen, die aus Kunststoff bestehen, ausgeführt. Die Wandungen sind an der Unterseite des Behälters derart miteinander verschweißt, dass ein talförmiger Bereich 19 entsteht. An der Oberseite des Behälters befindet sich ein Verbindungsmittel 12. Dies weist einen nach außen gerichteten ersten Verbinder 120 und einen ebenfalls nach außen In Figur 2 ist ein erfindungsgemäßer Behälter 10 dargestellt. Er ist als Innenbeutel 14 mit flexiblen Wandungen, die aus Kunststoff bestehen, ausgeführt. Die Wandungen sind an der Unterseite des Behälters derart miteinander verschweißt, dass ein talförmiger Bereich 19 entsteht. An der Oberseite des Behälters befindet sich ein Verbindungsmittel 12. Dies weist einen nach außen gerichteten ersten Verbinder 120 und einen ebenfalls nach außen gerichteten zweiten Verbinder 122 auf, mit denen der Behälter 10 an der Dialysemaschine montiert wird. Der erste Verbinder 120 bildet die Fluidverbindung mit dem Einführmittel 16, durch das Flüssigkeit und/oder Gas in den Behälter 10 eingeleitet werden. Das Einführmittel ragt in den unteren talförmigen Bereich 19 des Behälters 10, der teilweise mit Konzentrat 20 gefüllt ist. Durch diesen Aufbau wird die Auflösung des Trockenkonzentrates bei der Herstellung eines Flüssigkonzentrates durch ein Verfahren, wie in der DE 10 2011 106 248.7 beschrieben, optimiert. Der zweite Verbinder 122 bildet die Fluidverbindung zu dem Entlüftungsmittel 16, über welches Gas, das z.B. während der Ausführung des in der DE 10 2011 106 248.7 beschriebenen Verfahrens in den Behälter eingeleitet wird, aus dem Behälter in die Dialysemaschine abgeleitet werden kann. Der Innenbeutel 14 wird weitgehend umschlossen von einem Außenbeutel 22, der mit Luft gefüllt ist. In der Wandung 14 ist ein Rückschlagventil 24 vorgesehen, das den Innenbeutel 14 mit dem Außenbeutel 22 verbindet. So kann der Außenbeutel 22 mit Gas befüllt werden, das von der Dialysemaschine über den Verbinder 120 und das Einführmittel 16 in den Innenbeutel geleitet wird. Das Gas könnte aber auch von außen mittels eines im Außenbeutel angebrachten Ventils (nicht gezeigt) in den Behälter 10 eingebracht werden. Der Außenbeutel 22 ist hier in gefüllten Zustand dargestellt. Durch seine starre Form stützt er den flüssigkeitsgefüllten Innenbeutel 14. Der Außenbeutel 22 ist an der Unterseite gerade ausgebildet, wodurch der Behälter 10 selbststehend ist.

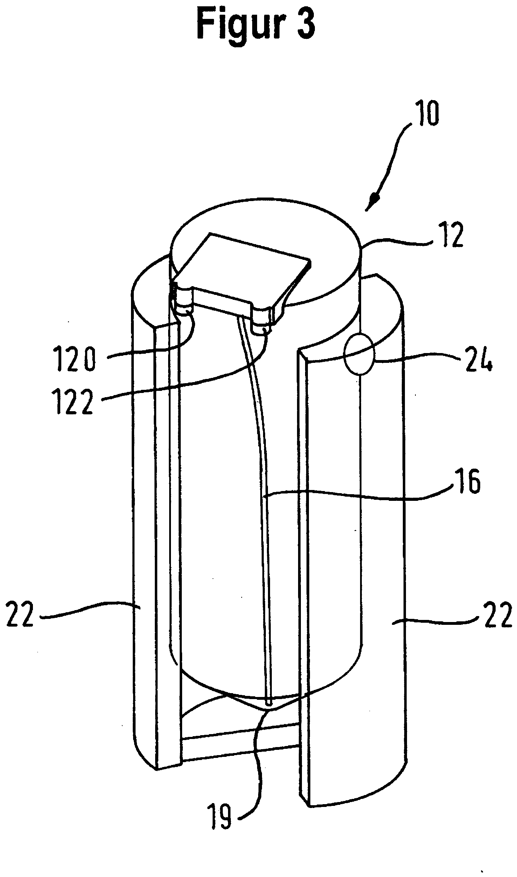

In Figur 3 wird durch die perspektivische Darstellung des Behälters 10 die Stützfunktion des Luftpolsters gezeigt.

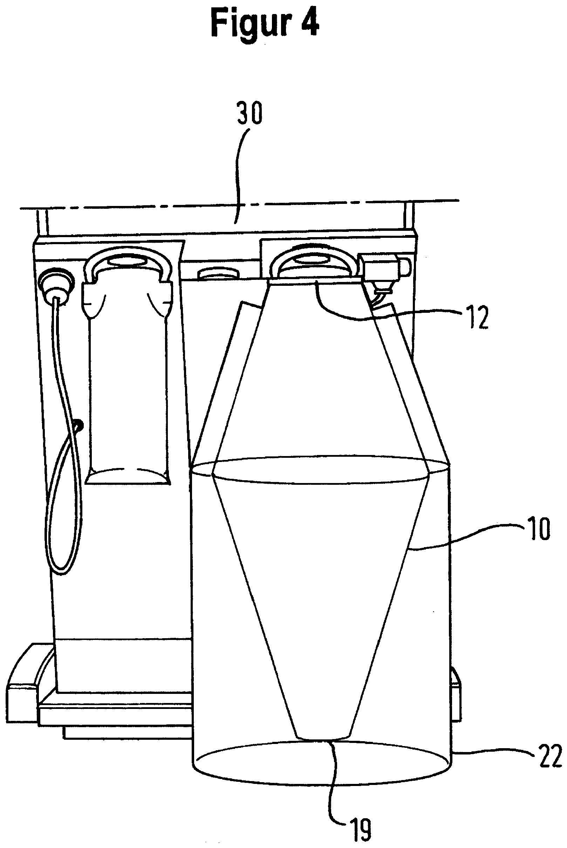

In Figur 4 ist der Behälter 10 der Säurekonzentrat enthält mit einer Dialysemaschine 30 verbunden. Da der Behälter 10 auf dem Boden steht, entsteht an der Konnektionsstelle der Dialysemaschine 30 keine Zugbelastung durch das Gewicht des Behälters.

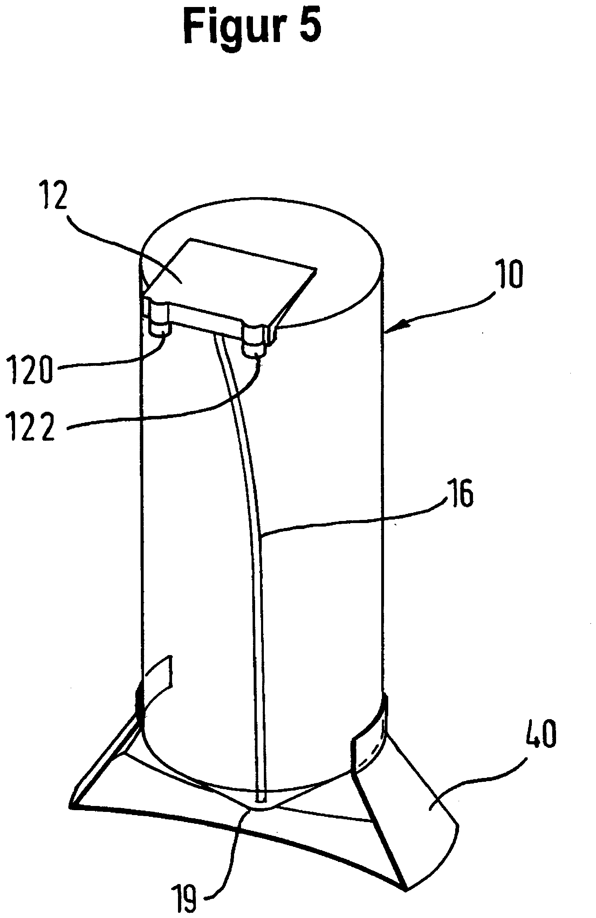

In Figur 5 ist ein erfindungsgemäßer Behälter 10 dargestellt, der durch einen Fuß 40 stabilisiert und selbststehend wird, der sich während des Befüllungsvorgangs entfaltet hat.

## Patentansprüche

1. Behälter (10) enthaltend ein Konzentrat (20), das zur Herstellung wenigstens einer Dialyselösung geeignet ist, der zumindest ein Verbindungsmittel (12) aufweist, mittels dessen der Behälter (10) an ein Dialysegerät (30) oder eine Zubereitungseinheit für Flüssigkonzentrate konnektierbar ist, **dadurch gekennzeichnet, dass** der Behälter (10) Luftpolster als integrierte Stützelemente (22) aufweist, die erst bei Zufuhr von Flüssigkeit und eines Gases durch die Dialysemaschine oder die Zubereitungseinheit in den Behälter ihre Stützfunktion ausprägen, und wobei das Luftpolster in Form eines Außenbeutels um einen Konzentrat-gefüllten Innenbeutel ausgebildet ist.

2. Behälter (10) nach Anspruch 1, **dadurch gekennzeichnet, dass** das Konzentrat (20) ein Trockenkonzentrat ist.

3. Behälter (10) nach Anspruch 1 oder 2, **dadurch gekennzeichnet, dass** Innen- (14) und Außenbeutel (22) über ein Rückschlagventil (24) verbunden sind.

4. Behälter (10) nach Anspruch1, 2, oder 3, **dadurch gekennzeichnet, dass** er ein Einführmittel (16) für Luft aufweist und ein Entlüftungsmittel (16') aufweist.

5. Behälter (10) nach einem der vorangehenden Ansprüche, **dadurch gekennzeichnet, dass** er nach der Ausprägung der Stützfunktion selbststehend ist.

6. Verwendung eines Behälters (10) nach einem der Ansprüche 1 bis 5, wobei der Behälter (10) zur Herstellung eines Konzentrates (20), insbesondere eines sauren Flüssigkonzentrates, verwendet wird, das seinerseits zur Herstellung einer Dialyselösung, vorzugsweise einer Dialyselösung für die Hämodialyse dient.

7. Dialysegerät (30) oder Zubereitungseinheit zur Herstellung eines Dialysekonzentrates (20), **dadurch gekennzeichnet, dass** das Dialysegerät (30) oder die Zubereitungseinheit mit einem Behälter (10) gemäß einem der Ansprüche 1 bis 5 konnektiert ist.

8. Dialysegerät (30) oder Zubereitungseinheit nach Anspruch 7, **dadurch gekennzeichnet, dass** das Dialysegerät (30) oder die Zubereitungseinheit Mittel aufweist, die derart ausgebildet sind, dass durch diese Mittel wenigstens eine Flüssigkeit, vorzugsweise Wasser, und wenigstens ein Gas, vorzugsweise Luft, in den mit dem Dialysegerät (30) oder der Zubereitungseinheit konnektierten Behälter (10) einleitbar ist, bzw. eingeleitet.

9. Dialysegerät (30) oder Zubereitungseinheit nach Anspruch 7 oder 8, **dadurch gekennzeichnet, dass** wenigstens eine Steuer- oder Regeleinheit vorgesehen ist, die derart ausgeführt ist, dass sie die Zufuhr von Flüssigkeit, vorzugsweise Wasser, und Gas, vorzugsweise Luft, in den Behälter (10) aus dem Behälter (10) steuert oder regelt.

10. Verfahren zur Bereitstellung eines stabilen Behälters (10) für Konzentrate (20) für die Dialyse durch
a) Konnektieren des Behälters (10) gemäß eines der Ansprüche 1 bis 5 an ein Dialysegerät (30) oder eine Zubereitungseinheit zur Herstellung von Dialysekonzentraten
b) Ausprägung der Stützfunktion durch Zufuhr einer Flüssigkeit, vorzugsweise Wasser, und eines Gases, vorzugsweise Luft, in den Behälter (10), wodurch durch die Zufuhr von Luft ein Luftpolster (22) gefüllt wird, das den Behälter (10) stabilisert.

## Claims

1. A container (10), containing a concentrate (20) suitable for production of at least one dialysis solution, comprising at least one connecting means (12), by which the container (10) can be connected to a dialysis machine (30) or a preparation unit for liquid concentrates, **characterized in that** the container (10) has air cushions as integrated supporting elements (22), which develop their supporting function only when a liquid and a gas are supplied to the container through the dialysis machine or the preparation unit, and wherein the air cushion is embodied in the form of an outer bag around a concentrate-filled inner bag.

2. The container (10) according to claim 1, **characterized in that** the concentrate (20) is a dry concentrate.

3. The container (10) according to claim 1 or 2, **characterized in that** the inner bag (14) and the outer bag (22) are connected by a nonreturn valve (24) .

4. The container (10) according to claim 1, 2 or 3, **characterized in that** it has insertion means (16) for air and has a ventilation means (16').

5. The container (10) according to any one of the preceding claims, **characterized in that** it is self-supporting, depending on the extent of the supporting function.

6. Use of a container (10) according to any one of claims 1 to 5, wherein the container (10) is used to prepare a concentrate (20), in particular an acidic liquid concentrate, which is in turn used to prepare a dialysis solution, preferably a dialysis solution for hemodialysis.

7. A dialysis machine (30) or preparation unit for preparing a dialysis concentrate (20), **characterized in that** the dialysis machine (30) or the preparation unit is connected to a container (10) according to any one of claims 1 to 5.

8. The dialysis machine (30) or preparation unit according to claim 7, **characterized in that** the dialysis machine (30) or the preparation unit comprises means that are embodied, so that through these means, at least one liquid, preferably water, and at least one gas, preferably air, are and/or can be introduced into the container (10) connected to the dialysis machine (30) or the preparation unit.

9. The dialysis machine (30) or preparation unit according to claim 7 or 8, **characterized in that** at least one control or regulating unit is provided and is embodied in such a way that it controls or regulates the supply of liquid, preferably water, and gas, preferably air, into the container (10) and out of the container (10).

10. A method for providing a stable container (10) for concentrates (20) for dialysis by
a) connecting the container (10) according to any one of claims 1 to 5 to a dialysis machine (30) or a preparation unit for preparing dialysis concentrates,
b) developing the supporting function by supplying a liquid, preferably water, and a gas, preferably air, to the container (10), so that an air cushion (22), which stabilizes the container (10), is filled by the supply of air.

## Revendications

1. Réservoir (10) contenant un concentré (20) qui est adapté pour la préparation d'au moins une solution de dialyse et qui présente au moins un moyen de connexion (12) au moyen duquel le réservoir (10) est connectable à un appareil de dialyse (30) ou à une unité de préparation pour des concentrés liquides, **caractérisé en ce que** le réservoir (10) présente des coussins d'air faisant office d'éléments d'appui intégrés (22) qui n'exercent leurs fonctions d'appui qu'en cas d'acheminement de liquide et d'un gaz par la machine de dialyse ou l'unité de préparation vers le réservoir, le coussin d'air étant réalisé sous forme d'une poche extérieure et d'une poche intérieure remplie de concentré.

2. Réservoir (10) selon la revendication 1, **caractérisé en ce que** le concentré (20) est un concentré sec.

3. Réservoir (10) selon la revendication 1 ou 2, **caractérisé en ce que** les poches intérieure (14) et extérieure (22) sont connectées par une soupape antiretour (24).

4. Réservoir (10) selon la revendication 1, 2 ou 3, **caractérisé en ce qu'**il comprend un moyen d'introduction (16) pour l'air et un moyen de purge d'air (16').

5. Réservoir (10) selon une des revendications précédentes, **caractérisé en ce qu'**il est autoporteur après l'exercice de la fonction d'appui.

6. Utilisation d'un réservoir (10) selon une des revendications 1 à 5, dans laquelle le réservoir (10) est utilisé pour préparer un concentré (20), en particulier un concentré liquide acide qui sert de son côté à préparer une solution de dialyse, de préférence une solution de dialyse pour hémodialyse.

7. Appareil de dialyse (30) ou unité de préparation d'un concentré de dialyse (20), **caractérisé en ce que** l'appareil de dialyse (30) ou l'unité de préparation est connecté à un réservoir (10) selon une des revendications 1 à 5.

8. Appareil de dialyse (30) ou unité de préparation selon la revendication 7, **caractérisé en ce que** l'appareil de dialyse (30) ou l'unité de préparation présente des moyens qui sont réalisés de manière à ce que, grâce à ces moyens, au moins un liquide, de préférence de l'eau, et au moins un gaz, de préférence de l'air, puisse être conduit ou soit conduit dans le réservoir (10) connecté à l'appareil de dialyse (30) ou à l'unité de préparation.

9. Appareil de dialyse (30) ou unité de préparation selon la revendication 7 ou 8, **caractérisé en ce qu'**il est prévu au moins une unité de commande ou de réglage qui est conçue pour commander ou régler l'acheminement de liquide, de préférence de l'eau, et de gaz, de préférence de l'air, vers le réservoir (10) en provenance du réservoir (10).

10. Procédé de mise à disposition d'un réservoir (10) pour des concentrés (20) destinés à la dialyse par
a) connexion du réservoir (10) selon une des revendications 1 à 5 à un appareil de dialyse (30) ou à une unité de préparation pour la préparation de concentrés de dialyse,
b) exercice de la fonction d'appui par acheminement d'un liquide, de préférence de l'eau, et d'un gaz, de préférence de l'air, dans le réservoir (10), ce qui a pour effet, du fait de l'acheminement d'air, de remplir un coussin d'air (22) qui stabilise le réservoir (10).
